(19) Europäisches Patentamt
European Patent Office
Office européen des brevets

(11) Veröffentlichungsnummer: **0 281 027 A2**

(12) # EUROPÄISCHE PATENTANMELDUNG

(21) Anmeldenummer: **88102883.1**

(51) Int. Cl.⁴: **A61K 7/50**, C11D 1/37

(22) Anmeldetag: **26.02.88**

(30) Priorität: **05.03.87 DE 3707014**

(43) Veröffentlichungstag der Anmeldung: **07.09.88 Patentblatt 88/36**

(84) Benannte Vertragsstaaten: **AT BE CH DE ES FR GB GR IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Tesmann, Holger, Dr.**
**Vennstrasse 61**
**D-4000 Düsseldorf 12(DE)**
Erfinder: **Wegener, Ingo**
**Am Falder 20**
**D-4000 Düsseldorf 13(DE)**
Erfinder: **Hensen, Hermann, Dr.**
**Tizianweg 4**
**D-4010 Hilden(DE)**
Erfinder: **Meffert, Alfred, Dr.**
**Marie-Curie-Strasse 10**
**D-4019 Monheim(DE)**

(54) **Wässrige Tensidzusammensetzungen mit Alpha-Sulfofettsäure-Salzen.**

(57) Wäßrige Tensidzubereitungen auf Basis von oberflächenaktiven Alkylethersulfaten, Alpha-Olefinsulfonaten, Sulfobernsteinsäuremonoestern und sekundären Alkansulfonaten oder deren Mischungen lassen sich bezüglich der Hautverträglichkeit und des Hautgefühls verbessern durch Zusatz von Alpha-Sulfofettsäure-Salzen der Formel

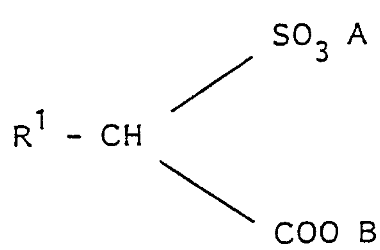

in der $R^1$ eine lineare $C_9$-$C_{21}$-Alkylgruppe, A ein Alkali-, Ammonium-oder Alkanolammonium-Kation und B Wasserstoff oder eine der Bedeutungen von A hat.

EP 0 281 027 A2

## "Wässrige Tensidzusammensetzungen mit Alpha-Sulfofettsäure-Salzen"

Gegenstand der Erfindung ist die Verbesserung der Hautverträglichkeit tensidhaltiger, wässriger Zubereitungen durch Zusatz von geringen Mengen von Alpha-Sulfofettsäure-Salzen.

Tensidhaltige Zubereitungen, die entweder bei der Anwendung mit der Haut in Berührung kommen oder die zur Reinigung und Pflege des Körpers, der Haut oder der Haare verwendet werden, weisen häufig den Nachteil auf, daß sie zu einer starken Entfettung der Haut und/oder einer unzuträglichen Quellung der Keratinschicht führen und dadurch die Haut in einen trockenen, spröden oder rauhen Zustand versetzen, der ein unangenehmes Hautgefühl verursacht. Es sind bereits viele Zusätze zur Rückfettung oder zur Hautbefeuchtung vorgeschlagen worden.

Aus US-PS-2,915,473 waren auch bereits Tensid-Zusammensetzungen bekannt, die mindestens 10 Gewichtsprozent eines Alpha-Sulfofettsäure-Salzes enthalten. Diese Kombination soll besonders effektive Wasch-und Reinigungswirkung aufweisen.

Es war bekannt, daß Alpha-Sulfofettsäure-Salze eine viskositätserniedrigende Wirkung in Waschaktivsubstanzkonzentraten auf Basis von Fettalkoholsulfaten (vgl. DE-PS-12 18 646), von oberflächenaktiven Alkylbenzolsulfonaten (vgl. DE-PS-12 16 740) und von Seifen (vgl. DE-PS-12 25 799) aufweisen. Es war weiterhin aus DE-AS 21 61 726 bekannt, daß Alpha-Sulfofettsäure-Salze in Mengen von 10 bis 75 Gewichtsprozent als Gerüststoffbestandteile in Waschmitteln geeignet sind. Außerdem bewirken Alpha-Sulfo fettsäure-Salze eine Verbesserung der Pulvereigenschaften von Waschpulvern auf Basis von Alkansulfonaten und Olefinsulfonaten (vgl. DE-PS-12 71 295) von nichtionogenen Tensiden (DE-PS-12 81 619) und Alpha-Sulfofettsäureestern (DE-PS-11 76 307, DE-PS-12 11 742 und DE-PS-11 87 758).

Es wurde nun aber gefunden, daß sich die Hautverträglichkeit und die hautkosmetischen Eigenschaften von tensidhaltigen wässrigen Zubereitungen merklich verbessern lassen, wenn man diesen relativ geringe Mengen von Alpha-Sulfofettsäure-Salzen zusetzt.

Gegenstand der Erfindung ist daher die Verwendung von Alpha-Sulfofettsäure-Salzen der Formel (I)

(I) 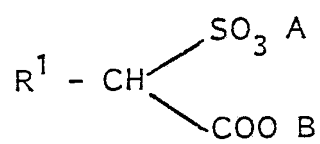

in der $R^1$ eine lineare Alkylgruppe mit 9 bis 21 C-Atomen, A ein Alkali-, Erdalkali-, Ammonium-, oder ein Mono-, Di-oder Trialkanolammoniumion mit 2 oder 3 C-Atomen in der Alkanolgruppe und B Wasserstoff ist oder eine der Bedeutungen von A hat, als Zusatz zur Verbesserung der Hautverträglichkeit und des Hautgefühls von wässrigen Tensidzusammensetzungen auf Basis von oberflächenaktiven Alkylethersulfaten, Alpha-Olefinsulfonaten, Sulfobernsteinsäuremonoestern und Alkansulfonaten oder deren Mischungen. Ein merklich verbessertes Hautgefühl wird bereits durch Zusätze von 0,1 bis 9 Gewichtsprozent der Alpha-Sulfofettsäure-Salze erreicht.

Es wurde auch gefunden, daß erfindungsgemäße tensidhaltige wässrige Zubereitungen, wenn sie zur Wäsche oder Pflege der Haare verwendet werden, diesen eine verbesserte Naßkämmbarkeit verleihen.

Alpha-Sulfofettsäure-Salze der Formel I sind literaturbekannt. Die Herstellung von Alpha-Sulfopalmitinsäure-di-Natriumsalz und dessen Homologen aus Laurin-, Myristin-und Stearinsäure sowie deren Eigenschaften werden z.B. in J. Am. Oil Chem. Soc., May 1952, Seite 198 bis 201 beschrieben. Auf analoge Weise lassen sich auch andere Alpha-Sulfofettsäure-Salze der Formel I, z.B. die Ammonium-und Alkanolammonium-Salze herstellen, wenn man zur Neutralisation die entsprechenden Basen verwendet. Geeignete Basen sind z.B. LiOH, NaOH, KOH, $Mg(OH)_2$, $NH_3$, Mono-Di-oder Triethanolamin oder Mono-, Di-oder Triisopropanolamin. Auch die Herstellung gemischter Salze, z.B. der Natrium-Triethanolammonium-Mischsalze kann analog erfolgen, indem man die Neutralisation mit 1 Mol Natriumhydroxid und 1 Mol Triethanolamin pro Mol Alpha-Sulfofettsäure durchführt. Auch "saure" Salze der Alpha-Sulfotettsäuren sind durch Neutralisation mit 1 Mol der Base herstellbar.

Oberflächenaktive Alkylethersulfate sind Verbindungen der Formel (II)

$$\text{(II)} \qquad R-(O\,C_n\,H_{2n})_x-OSO_3^{(-)}$$

in der $R^2$ eine lineare oder wenig verzweigte Alkylgruppe mit 6 bis 22 C-Atomen, n = 2 oder 3, und x = 1 bis 20 sein kann, in Form ihrer Alkali-, Magnesium-, Ammonium-, Mono-, Di-, oder Trialkanolammoniumsalze mit 2 oder 3 C-Atomen in der Alkanolgruppe.

Oberflächenaktive Alpha-Olefinsulfonate sind Tenside, die durch Sulfonierung von Alpha-Olefinen mit 8 bis 18 C-Atomen mit Schwefeltrioxid nach literaturbekannten Verfahren erhalten werden.

Oberflächenaktive Sulfobernsteinsäuremonoester werden durch Umsetzung von Maleinsäureanhydrid mit $C_8$-$C_{18}$-Fettalkoholen oder mit Anlagerungsprodukten von 1 bis 10 Mol Ethylenoxid an $C_8$-$C_{18}$-Fettalkohole zum Monoester und Umsetzung des Maleinsäuremonoesters mit Natriumsulfit oder Natriumbisulfit erhalten.

Oberflächenaktive sekundäre Alkansulfonate werden durch Sulfoxidation von linearen Alkanen mit 8 bis 18 C-Atomen mit $SO_2$ und Luft oder durch Sulfochlorierung der Alkane mit $SO_2$ und Chlor in Gegenwart von Ultraviolettlicht und anschließende Hydrolyse der Alkansulfochloride nach ebenfalls literaturbekannten Verfahren erhalten.

Die genannten oberflächenaktiven anionischen Tenside sind von besonderer Bedeutung für die Herstellung von flüssigen wässrigen Tensidzubereitungen für die Körperpflege oder für manuelle Spülmittel. Für solche Produkte ist die gefundene Verbesserung der Hautverträglichkeit und des Hautgefühls besonders vorteilhaft.

Ein weiterer Erfindungsgegenstand sind daher wässrige Tensidzusammensetzungen mit einem Gehalt an

(A) 3 bis 50 Gewichtsprozent Alkylethersulfaten der Formel II, in der $R^2$ eine lineare Alkylgruppe mit 6 bis 22 C-Atomen, n = 2 oder 3 und x = 1 bis 20 ist, Alpha-Olefinsulfonaten mit 8 bis 18 C-Atomen, sekundären Alkansulfonaten mit 8 bis 18 C-Atomen oder Mischungen dieser Tenside in Form der Alkali-, Magnesium-, Ammonium-, Mono-, Di-oder Trialkanolammoniumsalze mit 2 oder 3 C-Atomen in der Alkanolgruppe und die frei sind von Alpha-Sulfofettsäureestersalzen, gekennzeichnet durch einen Gehalt von

(B) 0,1 bis 9 Gewichtsprozent an Alpha-Sulfofettsäure-Salzen der Formel I.

Außer den genannten obligatorischen Tensidkomponenten können die erfindungsgemäßen Tensidzusammensetzungen weitere oberflächenaktive Verbindungen in unterschiedlichen Mengen enthalten. Solche weiteren Tenside sind z.B. andere anionische Tenside, z.B. die Alkali-, Ammonium-und Alkanolammoniumsalze linearer Fettsäuren mit 8 bis 18 C-Atomen, der Ethercarbonsäuren der allgemeinen Formel $R^3$-X-($CH_2$-$CH_2O)_y$ -$CH_2$-COOH, in der $R^3$ eine lineare oder verzweigte Alkylgruppe mit 6 bis 22 C-Atomen, X ein Sauerstoffatom oder eine NH-Gruppe und y = O oder eine ganze Zahl von 1 bis 8 ist, der Acylsarcosine, der Acylisethionate, der Acyltaurine mit jeweils 8 bis 18 C-Atomen in der Acylgruppe, der Alkyl($C_8$-$C_{18}$)-phosphate, der Alkyl ($C_8$-$C_{18}$)-glycerylsulfate, der Alkyl($C_8$-$C_{18}$)-phenolpolyglycolethersulfate mit 2 bis 8 Glycolethergruppen, der Fettsäure($C_{12}$-$C_{18}$)-monoethanolamidpolyglycolethersulfate mit 1 bis 6 Glycolethergruppen, der Sulfobernsteinsäuremono-und/oder diester von $C_8$-$C_{18}$-Fettalkoholen oder von $C_8$-$C_{18}$-Fettalkoholpolyglycolethern mit 2 bis 12 Glycolethergruppen.

Nicht zur Erfindung gehören Zubereitungen, die als Tenside Alpha-Sulfofettsäure-$C_1$-$C_4$-ester enthalten, die, bedingt durch das Herstellverfahren, bereits als Nebenprodukte 10 bis 20 Gewichtsprozent, bezogen auf den Alpha-Sulfofettsäureester, an Alpha-Sulfofettsäure-Salzen enthalten.

Weitere Tenside, die in den erfindungsgemäßen Zusammensetzungen zusätzlich enthalten sein können, sind die ampholytischen und zwitterionischen und die nichtionogenen Tenside. Unter ampholytischen Tensiden werden solche oberflächenaktiven Verbindungen verstanden, die außer einer $C_8$-$C_{18}$-Alkylgruppe eine freie Aminogruppe und eine -COOH-oder -$SO_3$H-Gruppe im Molekül tragen und zur Ausbildung innerer Salze befähigt sind. Zwitterionische Tenside tragen außer einer $C_8$-$C_{18}$-Alkylgruppe in ihrem Molekül eine quartäre Ammoniumgruppe und eine -$COO^{(-)}$-oder -$SO_3^{(-)}$-Gruppe. Beispiele für ampholytische Tenside sind z.B. die 2-Alkyl-($C_8$-$C_{18}$)-aminopropionsäure oder Alkyl-($C_8$-$C_{18}$)-aminoessigsäure. Beispiele für zwitterionische Tenside sind N-Alkyl-($C_8$-$C_{18}$)-dimethyl-ammonio-glycinat, N-Acyl-($C_8$-$C_{18}$)-aminopropyl-dimethyl-ammonio-glycinat, 2-Alkyl-($C_8$-$C_{18}$)-3-carboxymethyl-3-hydroxyethyl-imidazolin. Nichtionische Tenside sind z.B. die Anlagerungsprodukte von 1 bis 30 Mol Ethylenoxid oder Propylenoxid an Fettalkohole mit 8 bis 22 C-Atomen, an $C_{12}$-$C_{18}$-Fettsäuren, an $C_{12}$-$C_{18}$-Fettamine, an Alkylphenole mit 8 bis 15 C-Atomen in der Alkylgruppe, an Fettsäure-($C_{12}$-$C_{18}$)-monoethanolamide, an Fettsäure ($C_{12}$-$C_{18}$)-mono-und -diglyceride und an Fettsäure ($C_{12}$-$C_{18}$)sorbitan-partialester. Weitere geeignete nichtionische Tenside sind Zucker-Fettsäureester, Alkylglucoside und Alkyloligoglucoside mit 8 bis 18 C-Atomen in der Alkylgruppe und Methylglucosid-Fettsäurepartialester sowie deren Ethylenoxidanlagerungsprodukte und die Glycerin-oxethylat-Fettsäureester. Schließlich gehören auch die tertiären Aminoxid-Tenside zu den geeigneten nichtionischen Tensiden.

Besonders bevorzugt sind erfindungsgemäße Tensidzusammensetzungen, die zusätzlich

(C) 0,1 bis 10 Gewichtsprozent eines Fettsäure ($C_{12}$-$C_{18}$)-mono-oder -dialkanolamids mit 2 oder 3 C-Atomen in der Alkanolgruppe enthalten.

Von den anionischen Tensiden kommt den Alkylethersulfaten wegen ihrer guten Schäumeigenschaften, Härteunempfindlichkeit und Verdickbarkeit mit Fettsäurealkanolamiden und Kochsalz die größte Bedeutung zu. Eine besonders bevorzugte Ausführung der Erfindung sind daher Tensidzusammensetzungen, die (A) 3 bis 50 Gewichtsprozent Alkylethersulfate der Formel II, in der R eine Alkylgruppe mit 12 bis 16 C-Atomen, n = 2 und x = 1 - 6 ist, enthalten sind.

Die Alpha-Sulfofettsäure-Alkalisalze, insbesondere die Di-Natriumsalze und die Di-Kaliumsalze sind in Wasser nur begrenzt löslich. Erfindungsgemäße Zusammensetzungen, die diese Salze enthalten, erscheinen daher leicht getrübt und es kann empfohlen werden, zur Verbesserung des Aussehens und der Lagerstabilität Trübungs-und Perlglanzmittel sowie Verdickungsmittel und dispersionsstabilisierende Zusätze zuzufügen. Besonders geeignet sind hierfür Perlglanzkonzentrate, wie sie z.B. aus DE-A-16 69 152 oder aus DE-A-35 19 080 bekannt sind.

Klarflüssige wässrige Tensidzusammensetzungen gemäß der Erfindung lassen sich am besten dadurch erhalten, daß man das Alpha-Sulfofettsäuresalz der Formel I als Mono-oder Di-Salz eines Mono-, Di-oder Trialkanolamins mit 2 oder 3 C-Atomen in der Alkanolgruppe einsetzt. Geeignete Alkanolamine sind insbesondere Mono-, Di-und Triethanolamin sowie Mono-, Di-und Triisopropanolamin.

Neben den genannten Tensidkomponenten können die erfindungsgemäßen Tensidzusammensetzungen weitere bekannte Hilfs-und Zusatzmittel enthalten, die für die Herstellung von manuellen Spülmitteln und Reinigungsmitteln oder für die Herstellung kosmetischer Haut-und Haarreinigungsmittel geeignet sind z.B.

- kosmetische Öl-und Fettkomponenten, z.B. natürliche Öle oder synthetische Ester wie Isopropylmyristat, Decyloleat, Glycerintricaprylat, Fettalkohole wie z.B. Cetylalkohol, Oleylalkohol, 2-Octyl-dodecanol, natürliche und synthetische Wachse, Paraffine oder Silikone,
- Duftstoffe, ätherische Öle
- antimikrobielle Stoffe und Konservierungsmittel
- Verdickungsmittel, z.B. wasserlösliche Polymere wie Carboxymethylcellulose, Hydroxyethylcellulose, Polyacrylate, Polyvinylalkohol, Polyvinylpyrrolidon
- Komplexbildner
- Trübungs-und Perlglanzmittel
- Farbstoffe
- haarkosmetische Wirkstoffe, z.B. antistatische und haaravivierende Komponenten wie z.B. quartäre Ammoniumverbindungen und kationische Polymere mit quartären Ammoniumgruppen.

Die nachfolgenden Beispiele sollen den Erfindungsgegenstand weiter erläutern ohne ihn hierauf zu beschränken.

<u>Beispiele</u>

1. Prüfung der verbesserten Naßkämmbarkeit

Es wurden folgende Prüfshampoos hergestellt.

| | 1 | 2 | 3 | 4 | 5 | 6 |
|---|---|---|---|---|---|---|
| Natriumlaurylethersulfat ($C_{12/14}$ + 2 EO-sulfat, Na) | 15 | 14,5 | - | - | - | - |
| Magnesiumlaurylether-sulfat (Gemisch aus $C_{12/18}$ + 10 EO-sulfat und $C_{12/18}$ + 2 EO-sulfat) | - | - | 15 | 14,5 | 13 | 10 |
| Laurin-/Myristinsäure-sulfonat-di-Na-Salz | - | 0,5 | - | 0,5 | 2,0 | 5,0 |
| Wasser | 85 | 85 | 85 | 85 | 85 | 85 |
| Mittlere Naßkämmbarkeit NK (%) | 100 % | 64 % | 81 % | 65 % | 78 % | 76 % |

11 cm lange, 1 Gramm schwere braune, europäische, ungeschädigte Haarsträhnen wurden mit ca. 5 g der Prüfshampoos 5 Minuten bei 30 °C behandelt, anschließend mit lauwarmen Wasser gespült und überschüssiges Wasser abgestreift. Dann erfolgte die Messung des Kämmwiderstandes analog der in J. Soc. Cosmet. Chem. 27, 379-398 (September 1976) von M.L. Garcia und J. Diaz angege benen Methode. Dabei wurden alle Werte in Form von Paardifferenzen ermittelt, d.h. es wurde jeweils die Prüfsträhne mit der Strähne verglichen, die mit dem Prüfshampoo 1 (Blindwert ohne Fettsäure-Sulfonat) behandelt worden war. Alle Werte wurden an 20 Strähnen ermittelt und die Mittelwerte gebildet. Die mittlere Naßkämmbarkeit (NK %) ergibt sich dann aus

$$NK\ (\%) = \frac{\text{mittleres Arbeitsintegral Prüfshampoo}}{\text{mittleres Arbeitsintegral Blindwert}} \cdot 100$$

Werte unter 100 % bedeuten daher eine Verbesserung der Naßkämmbarkeit. Die Ergebnisse zeigen, daß insbesondere sehr niedrige Zusätze von Fettsäure-Sulfonat eine starke Verbesserung der Naßkämmbarkeit bewirken.

2. Rezepturbeispiele

1. Shampoo, perlglänzend Natrium-laurylethersulfat ($C_{12/14}$ + 2 EO-sulfat, Na) 9,0 Gew.%
Kokosfettsäure ($C_{12}$-$C_{18}$)-sulfonat, di-Na-Salz 4,0 Gew.%
Perlglanzkonzentrat (1) 3,0 Gew.%
Kokosfettsäurediethanolamid 2,0 Gew.%
5-Brom-5-nitro-1.3-dioxan (10 %ige Lösung in 1,2-Propylglycol) 0,2 Gew.%
Natriumchlorid 2,0 Gew.%
Parfümöl 0,3 Gew.%
Farbstoff (blau) 0,1 Gew.%
Citronensäure ad pH = 6,5
Wasser ad 100,0 Gew.%

2. Shampoo, klar flüssig Natrium-laurylethersulfat ($C_{12/14}$ + 2 EO-sulfat, Na) 10,0 Gew.%
Kokosfettsäure ($C_{12-18}$)-sulfonat, di-TEA-Salz 2,0 Gew.%
Kokosfettsäurediethanolamid 3,0 Gew.%
Natriumchlorid 2,5 Gew.%
5-Brom-5-nitro-1,4-dioxan (10 %ige Lösung in 1,2-Propylenglycol) 0,2 Gew.%
Parfümöl 0,2 Gew.%
Farbstoff (blau) 0,1 Gew.%
Citronensäure ad pH = 6,5
Wasser ad 100,0 Gew.%

3. Duschbad, perlglänzend Natriumlaurylethersulfat ($C_{12/14}$ + 2 EO-sulfat, Na) 6,0 Gew.%
Kokosfettsäure ($C_{12/18}$)-sulfonat, di-Na-Salz 6,0 Gew.%
perlglänzendes Tensidkonzentrat (2) 30,0 Gew.%
Kokosfettsäurediethanolamid 2,0 Gew.%
Glycerinoxethylat (7 EO)-kokosfettsäure ($C_{12/18}$)-ester 5,0 Gew.%
5-Brom-5-nitro-1,3-dioxan (10 %ige Lösung in 1,2-Propylenglycol) 0,2 Gew.%
Parfüm 0,3 Gew.%
Farbstoff 0,1 Gew.%
Citronensäure ad pH = 6,5
Wasser ad 100,0 Gew.%

4. Duschbad klarflüssig Natriumlaurylethersulfat ($C_{12/14}$ + 3 EO-sulfat, Na) 12,0 Gew.%
Kokosfettsäure ($C_{12/14}$)-sulfonat, di-TEA-Salz 1,0 Gew.%
N-Kokosfettacyl-aminopropyl-N,N-dimethyl-glycin 10,0 Gew.%
Kokosfettsäurediethanolamid 3,0 Gew.%
Parfüm 1,0 Gew.%
5-Brom-5-nitro-1,3-dioxan (10 %ige Lösung in 1,2-Propylenglycol) 0,2 Gew.%
Farbstoff (blau) 0,1 Gew.%
Wasser ad 100,0 Gew.%

(1) Das Perlglanzkonzentrat enthielt:
14 Gewichtsprozent Natriumlaurylethersulfat, 14 Gewichtsprozent Ethylenglycoldistearat, 6 Gewichtsprozent Kokosfettsä uremonoethanolamid und 3 Gewichtsprozent Kokosfettalkoholpolyglycolether (10 EO), Rest Wasser.

(2) Das perlglä nzende Tensidkonzentrat enthielt:
15 Gewichtsprozent Natrium-laurylsulfat, 12 Gewichtsprozent Natriumlaurylethersulfat, 3 Gewichtsprozent Laurinsä ureisopropanolamid, 2 Gewichtsprozent Kokosfettsä uremonoethanolamid, 4 Gewichtsprozent Ethylenglycoldistearat, 1 Gewichtsprozent Kokosfettalkoholpolyglycolether (10 EO), Rest Wasser.

5. Schaumbad Natriumlaurylethersulfat ($C_{12/14}$ + 2 EO-sulfat, Na) 21,0 Gew.%
Kokosfettsäure ($C_{12-18}$)-sulfonat, di-Na-Salz 5,0 Gew.%
Perlglanzkonzentrat (3) 15,0 Gew.%
Natriumchlorid 4,0 Gew.%
Parfüm 1,0 Gew.%
5-Brom-5-nitro-1,3-dioxan (10 %ige Lösung in 1,2-Propylenglycol) 0,2 Gew.%
Wasser ad 100,0 Gew.%

6. Manuelles Geschirrspülmittel Natrium-$C_{12}$-$C_{18}$-Alkansulfonat 10,0 Gew.%
Natrium-laurylethersulfat ($C_{12/14}$ + 2 EO-sulfat, Na) 7,0 Gew.%
Ethylenglycoldistearat 1,0 Gew.%
Kokosfettsäuremonoethanolamid 2,0 Gew.%
Kokosfettsäure-($C_{12}$-$C_{18}$)-sulfonat-di-Na-Salz 3,0 Gew.%
Harnstoff 5,0 Gew.%
Ethanol 3,0 Gew.%
Wasser 69,0 Gew.%

7. Allzweckreiniger Natrium-$C_{15}$-$C_{18}$-Olefinsulfonat 5,0 Gew.%
Nonylphenolpoly (10 EO)-glycolether 2,0 Gew.%
Kaliumpyrophosphat 5,0 Gew.%
Kokosfettsäure ($C_{12}$-$C_{18}$)-sulfonat-di-Triethanol-ammoniumsalz 2,0 Gew.%
Ammoniak (25 %ige wässrige Lösung) 1,0 Gew.%
Duftstoff 0,2 Gew.%
Wasser 84,8 Gew.%

## Ansprüche

1. Verwendung von Alpha-Sulfofettsäure-Salzen der Formel I

(I) 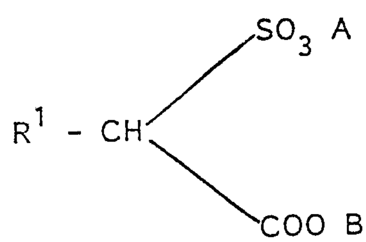

in der $R^1$ eine lineare Alkylgruppe mit 9 bis 21 C-Atomen, A ein Alkali-, Erdalkali-, Ammonium-, oder ein Mono-, Di-oder Trialkanolammoniumion mit 2 oder 3 C-Atomen in der Alkanolgruppe und B Wasserstoff ist oder eine der Bedeutungen von A hat, als Zusatz zur Verbesserung der Hautverträglichkeit und des Hautgefühls von wässrigen Tensidzusammensetzungen auf Basis von oberflächenaktiven Alkylethersulfaten, Alpha-Olefinsulfonaten, Sulfobernsteinsäuremonoestern und sekundären Alkansulfonaten oder deren Mischungen.

2. Wäßrige Tensidzusammensetzungen mit einem Gehalt an
(A) 3 bis 50 Gew.-% Alkylethersulfaten der Formel (II)

(II) $R^2$ -(O Cn $H_{2n})_x$-$OSO_3^{(-)}$

(3) Das Perlglanzkonzentrat enthielt:
21 Gewichtsprozent Natriumlaurylethersulfat, 20 Gewichtsprozent Ethylenglycoldistearat, 3 Gewichtsprozent Kokosfettsä uremonoethanolamid, 2 Gewichtsprozent Kokosfettalkoholpolyglycolether (10 EO), Rest Wasser.

in der $R^2$ eine lineare Alkylgruppe mit 8 bis 22 C-Atomen, n = 2 oder 3, und x = 1 bis 20 ist, Alpha-Olefinsulfonaten mit 8 bis 18 C-Atomen, sekundären Alkansulfonaten mit 12 bis 18 C-Atomen oder Mischungen davon in Form der Alkali-, Magnesium-, Ammonium-oder Mono-, Di-oder Trialkanolammoniumsalze mit 2 oder 3 C-Atomen in der Alkanolgruppe

und die frei sind von Alpha-Sulfofettsäureestersalzen, gekennzeichnet durch einen Gehalt von

(B) 0,1 bis 9 Gew.-% an Alpha-Sulfofettsäuresalzen der Formel (I) gemäß Anspruch 1.

3. Wäßrige Tensidzusammensetzungen nach Anspruch 2, dadurch gekennzeichnet, daß zusätzlich

(C) 0,1 bis 10 Gew.-% eines Fettsäure-$C_{12}$-$C_{18}$-mono-oder dialkanolamids mit 2 oder 3 C-Atomen in der Alkanolgruppe enthalten sind.

4. Wäßrige Tensidzusammensetzungen nach den Ansprüchen 2 oder 3, dadurch gekennzeichnet, daß

(A) 3 bis 50 Gew.-% Alkylethersulfate der Formel (II), in der R eine Alkylgruppe mit 12 bis 16 C-Atomen, n = 2 und x = 1 bis 6 ist, enthalten sind.

5. Klarflüssige wäßrige Tensidzusammensetzungen nach den Ansprüchen 2 bis 4, dadurch gekennzeichnet, daß das Alpha-Sulfofettsäuresalz der Formel (I) als Mono-oder Di-Salz eines Mono-, Di-oder Trialkanolamins mit 2 oder 3 C-Atomen in der Alkanolgruppe enthalten ist.